# EUROPEAN PATENT APPLICATION

(11) **EP 3 742 450 A1**
(43) Date of publication of application: **25.11.2020**
(21) Application number: 19215324.5
(22) Date of filing: 11.12.2019
(51) Int. Cl.: G16H 20/30, G09B 5/06, G09B 19/00

(54) **METHOD OF AUTOMATICALLY RECORDING COSMETOLOGY PROCEDURE**

(30) Priority: 24.05.2019 TW 108118048
(71) Applicant: CAL-COMP BIG DATA, INC, Shenkeng, New Taipei City 222 (TW)
(72) Inventor: CHEN, Ying-Yu, 222 NEW TAIPEI CITY (TW); HUANG, Yuan-Peng, 222 NEW TAIPEI CITY (TW)
(74) Representative: 2K Patentanwälte Blasberg Kewitz & Reichel

(57) **Abstract**

A method of automatically recording cosmetology procedure applied to a system of automatically recording cosmetology procedure is to capture detection images continuously, recognize a cosmetic product and start to measure time when determining that a user starts to use the cosmetic product, stop measuring time and record a usage of the cosmetic product in this round when determining that the user stops using the cosmetic product, and output a reminder notification of incorrect procedure when determining that there is incorrect usage of the cosmetic product according to a standard cosmetology procedure. The present disclosed example can record the usage of the cosmetic products automatically, and prevent the user from the incorrect usage of the cosmetic product.

## Description

### BACKGROUND OF THE INVENTION

### Field of the Invention

The technical field relates to automatic recording and more particularly related to a method of automatically recording cosmetology procedure.

### Description of Related Art

With the increase of cosmetology knowledge, people know that alternately using the multiple types of cosmetic products can maximize the effect of cosmetology, and no longer be limited to use single cosmetic product.

In general, people usually buy a plurality of different cosmetic products for alternate usage. However, because the ways (such as a usage order or a usage timing) to use each cosmetic product are different from each other, people usually forget the correct way to use the cosmetic product. Once the usage is incorrect, the cosmetology effect will reduce greatly.

Besides, people usually don't have time to record usage time of each cosmetic product. The above status makes people be unable to estimate the consumption rate of each cosmetic product based on the usage time after makeup, and make it usually happen that people find that the cosmetic product is run out during the cosmetology procedure and is unable to continue to finish the cosmetology procedure.

Accordingly, there is currently a need for a schema of automatically recording the usage of each cosmetic product used by the user, and actively reminding the user when the usage of any cosmetic product is incorrect.

### SUMMARY OF THE INVENTION

A method of automatically recording cosmetology procedure is disclosed in the present disclosed example, the method of automatically recording cosmetology procedure can record the usage of each cosmetic product used by the user automatically during makeup, and remind the user when the usage is incorrect.

One of the exemplary embodiments, a method of automatically recording cosmetology procedure, the method is applied to a system of automatically recording cosmetology procedure, the system of automatically recording cosmetology procedure comprises an image capture module, a display module and a processing module, the method of automatically recording cosmetology procedure comprises following steps: a) capturing detection images continuously by the image capture module under a record mode; b) recognizing a cosmetic product, retrieving cosmetic product data corresponding to the cosmetic product being recognized and starting to measure a single round usage time when determining that a user starts to use the cosmetic product based on the detection images by the processing module; c) stopping measuring the single round usage time and recording usage of the cosmetic product in this round when determining that the user stops using the cosmetic product based on the detection images; d) outputting a reminder notification of incorrect procedure by the display module when determining that there is incorrect usage of the cosmetic product based on a standard cosmetology procedure; and, e) performing the step b) to the step e) repeatedly until leaving from the record mode.

The present disclosed example can record the usage of the cosmetic products automatically, and prevent the user from the incorrect usage of the cosmetic product.

### BRIEF DESCRIPTION OF DRAWINGS

The features of the present disclosed example believed to be novel are set forth with particularity in the appended claims. The present disclosed example itself, however, may be best understood by reference to the following detailed description of the present disclosed example, which describes an exemplary embodiment of the present disclosed example, taken in conjunction with the accompanying drawings, in which:
FIG. 1 is an architecture diagram of a system of automatically recording cosmetology procedure according to one embodiment of the present disclosed example;
FIG. 2A is a usage schematic view of an electronic apparatus according to one embodiment of the present disclosed example;
FIG. 2B is a usage schematic view of an electronic apparatus according to one embodiment of the present disclosed example;
FIG. 3 is a flowchart of a method of automatically recording cosmetology procedure according to a first embodiment of the present disclosed example;
FIG. 4 is a partial flowchart of a method of automatically recording cosmetology procedure according to a second embodiment of the present disclosed example;
FIG. 5 is a flowchart of adding cosmetic product data of a third embodiment of the present disclosed example; and
FIG. 6 is a flowchart of building standard cosmetology procedure and running out reminding of a fourth embodiment of the present disclosed example.

### DETAILED DESCRIPTION OF THE INVENTION

In cooperation with attached drawings, the technical contents and detailed description of the present disclosed example are described thereinafter according to a preferable embodiment, being not used to limit its executing scope. Any equivalent variation and modification made according to appended claims is all covered by the claims claimed by the present disclosed example.

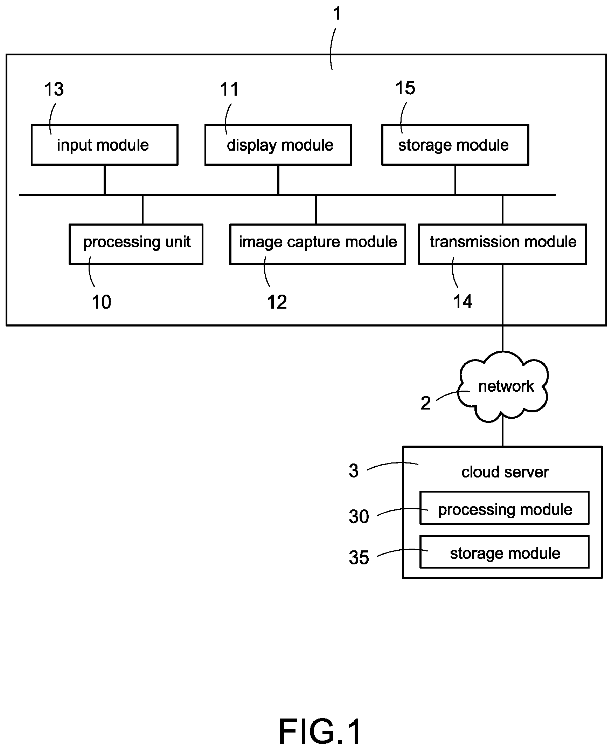

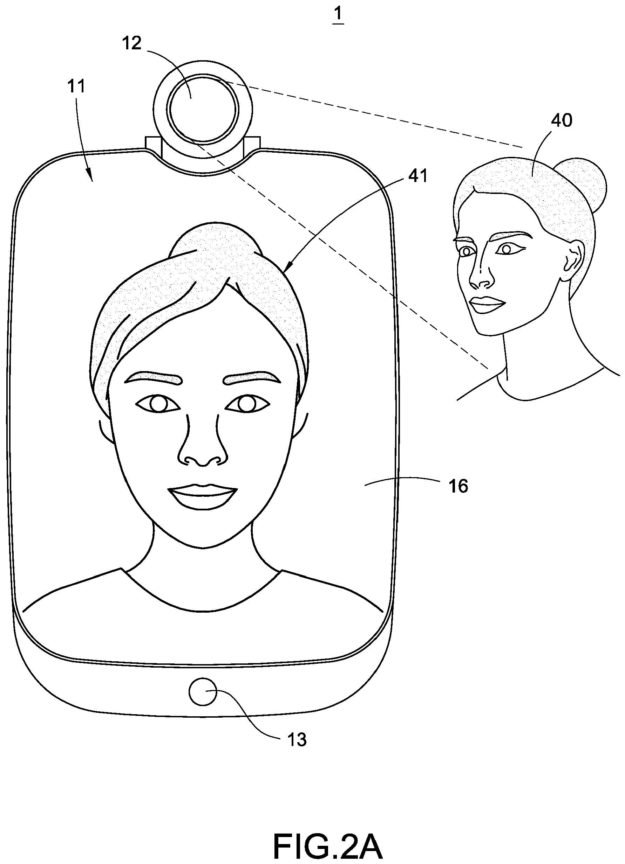

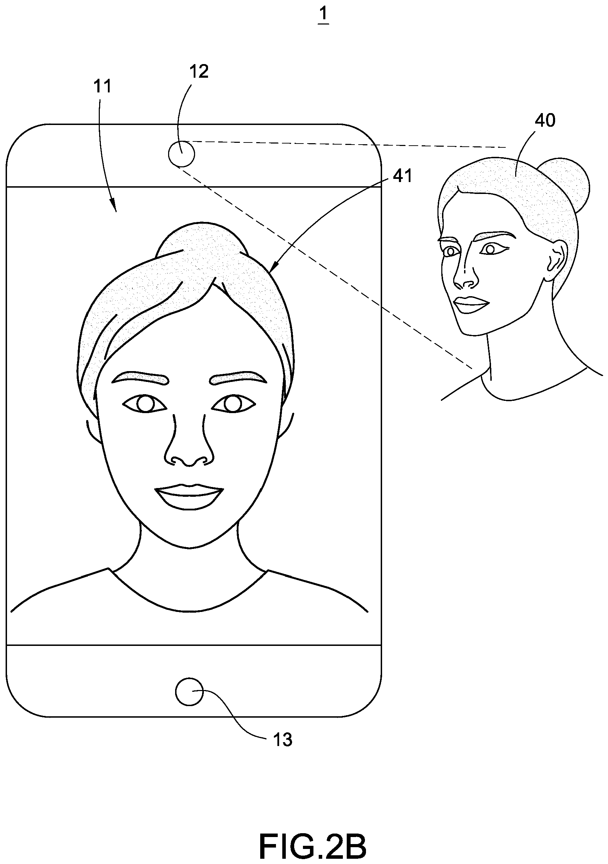

Please refer to FIG. 1, FIG. 2A and FIG. 2B together. FIG. 1 is an architecture diagram of a system of automatically recording cosmetology procedure according to one embodiment of the present disclosed example, FIG. 2A is a usage schematic view of an electronic apparatus according to one embodiment of the present disclosed example, and FIG. 2B is a usage schematic view of an electronic apparatus according to one embodiment of the present disclosed example.

A system of automatically recording cosmetology procedure (refer to as the automatic recording system in following description) is disclosed by the present disclosed example. Above automatic recording system be mainly used to execute a method of automatically recording cosmetology procedure (refer to as the automatic recording method in following description), and has the ability to automatically recognize the cosmetic product which the user currently uses during cosmetology procedure and record the usage time of each cosmetic product. Moreover, the automatic recording method can further remind the user if the usage of any cosmetic product is incorrect.

As shown in FIG. 1, the automatic recording system of the present disclosed example comprises an electronic apparatus 1. The electronic apparatus 1 mainly comprises a processing module 10, a display module 11, an image capture module 12, an input module 13, a transmission module 14 and a storage module 15. The processing module 10, the display module 11, the image capture module 12, the input module 13, the transmission module 14 and the storage module 15 are electrically connected to each other by at least one bus.

The display module 11 (such as color LCD monitor) is used to display information. The image capture module 12 (such as camera) is used to capture images. The input module 13 (such as buttons or touch pad) is used to receive the user operation. The transmission module 14 (such as Wi-Fi module, Bluetooth module, mobile network module or the other wireless transmission modules, or USB module, RJ-45 network module or the other wired transmission modules) is used to connect to the network 2 and/or the external apparatus. The storage module 15 is used to store data. The processing module 10 is used to control each device of the electronic apparatus 1 to operate.

One of the exemplary embodiments, the storage module 15 may comprise a non-transient storage media, the non-transient storage media stores a computer program (such as firmware, operating system, application program or any combination of the above program of the electronic apparatus 1), the computer program records a plurality of corresponding computer-executable codes. The processing module 10 may further implement the method of each embodiment of the present disclosed example via the execution of the computer-executable codes.

One of the exemplary embodiments, the automatic recording method of each embodiment of the present disclosed example is implemented in the local end. Namely, each embodiment of the present disclosed example may be implemented by the electronic apparatus 1 completely, but this specific example is not intended to limit the scope of the present disclosed example.

One of the exemplary embodiments, the automatic recording method of each embodiment of the present disclosed example may be implemented by combining with the cloud computing technology. More specifically, the transmission module 14 of the electronic apparatus 1 may be connected to the cloud server 3 via network 2, the cloud server 3 comprises a processing module 30 and a storage module 35. The automatic recording method of each embodiment of the present disclosed example may be implemented by making the cloud server 3 interact with the electronic apparatus 1.

One of the exemplary embodiments, as shown in FIG. 2A, the electronic apparatus 1 may be a smart mirror, and have the ability to provide the functions of optical mirror and electronic mirror simultaneously. More specifically, the electronic apparatus 1 may further comprise a mirror glass 16 (such as unidirectional glass) and a case. The mirror glass 16 is used for appearing an optical image 41 of the user 40 in reflection to implement the function of optical mirror. Above modules 10-15 may be arranged in the case of the electronic apparatus 1.

Furthermore, the display module 11 is arranged in the case and on the rear of the mirror glass 16. Namely, the user doesn't have the ability to discover the existence of the display module 11 directly by watching the appearance. Moreover, the display module 11 may display information on the mirror glass 16 by transmission after being turned on or the brightness of backlight being increased.

Furthermore, the processing module 10 may control the display module 11 to display the additional information (such as weather information, date information, graphical user interface or the other information) in the designated region, such as the edge of the mirror glass 16 or the other region having a lower probability of overlapping the optical mirror image 41.

Furthermore, the image capture module 12 may be arranged upon the mirror glass 18 and shoot toward the front of the mirror glass 16, so as to implement the electronic mirror function. The input module 13 may comprise at least one physical button arranged on the front side of the electronic apparatus 1, but this specific example is not intended to limit the scope of the present disclosed example.

Please be noted that the image capture module 12 is arranged upon the mirror glass 16, but this specific example is not intended to limit the scope of the present disclosed example. The image capture module 12 may be arranged in any position of the electronic apparatus 1 according to the product demand, such as being arranged behind the mirror glass 16 for reducing the probability of the image capture module 12 being destroyed and making the appearance simple.

One of the exemplary embodiments, as shown in FIG. 2B, the electronic apparatus 1 may be a general-purpose computer device (such as a smartphone, a tablet, or an electronic signboard with a camera function, take smartphone for example in FIG 2B), and only have the ability to provide a function of electronic mirror.

More specifically, the above-mentioned modules 10-15 may be installed in a case of the electronic apparatus 1, the image capture module 12 and the display module 11 may be installed on the same side(surface) of the electronic apparatus 1, so as to make the user be captured and watch the display module 11 simultaneously. Moreover, the electronic apparatus 1 may continuously capture images of the area in front of the electronic apparatus 1 (such as a facial image of the user) by the image capture module 12 when the execution of the computer program (such as the application program), execute the electable process(es) on the captured images optionally (such as the mirroring flip process or the brightness-adjusting process and so forth), and display the captured (processed) images by the display module 11 instantly. Thus, the user 40 may watch his/her electronic mirror image 41 on the display module 11.

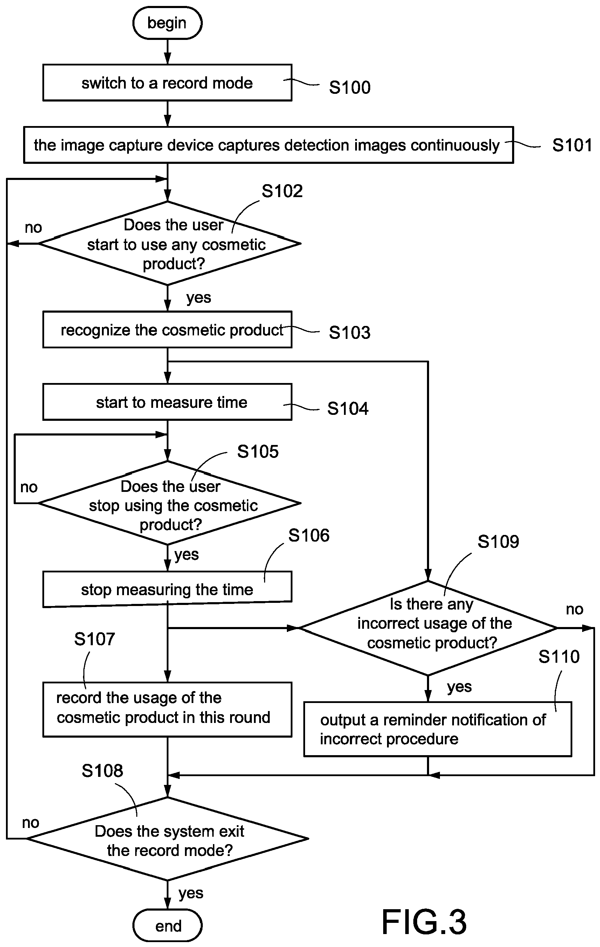

Please refer to FIG. 3, which is a flowchart of a method of automatically recording cosmetology procedure according to a first embodiment of the present disclosed example. The method of automatically recording cosmetology procedure of each embodiment of the present disclosed example may be implemented by the system of automatically recording cosmetology procedure shown in Fig. 1, FIG. 2A and Fig. 2B. The method of automatic recording method mainly comprises following steps.

Step: S100: the processing module 10 controls the electronic apparatus 1 to switch to a record mode. More specifically, when the user would like to start to execute cosmetology, the user may operate the electronic apparatus 1 to switch to the record mode, or the electronic apparatus 1 may switch to the record mode automatically by automatic detection. Moreover, under the record mode, the processing module 10 may control the electronic apparatus 1 to implement a record function described later by performing steps S102-S107.

One of the exemplary embodiments, the user may input a start cosmetology operation (such as pressing a record button of the input module 13) or a start cosmetology voice (such as the user speaking a voice "start cosmetology" if the input module 13 is a microphone) by the input module 13, or pose a start cosmetology gesture (such as the "1" gesture) to make the image capture module 12 capture the start cosmetology gesture. Then, the processing module 10 controls the electronic apparatus 1 to switch to the record mode when detecting the above designated input.

Step: S101: the processing module 10 shoots toward the front of the display module 11 by the image capture module 12 continuously for capturing the detection images comprising the image of the user continuously.

Step: S 102: the processing module 10 recognizes the image of the user in the newly captured detection images, and detects whether the user start to use any of the cosmetic product continuously.

One of the exemplary embodiments, the processing module 10 is configured to execute a user detection for detecting the position(s) of the designated portion(s), such as the face or the hand(s), of the user on the detection images, and simultaneously execute a cosmetic product detection for detecting the position(s) of all of the cosmetic product(s) enabling to be recognized in the screen. Then, the processing module 10 may determine whether the user starts to use any cosmetic product by comparing the position(s) of the designated portion(s) with the position(s) of the cosmetic products. For example, the user holds any cosmetic product continuously in his/her hand or any cosmetic product is very close to the face of the user.

If the processing module 10 determines that the user start to use any cosmetic product, the step S103 will be performed. Otherwise, the processing module 10 performs the step S102 again for continuous detection.

Step: S103: the processing module 10 further recognizes the cosmetic product which the user is currently using for determining the corresponding cosmetic product data. Namely, the processing module 10 retrieves data of the cosmetic product which the user is currently using.

One of the exemplary embodiments, the user may build a cosmetic cabinet list in the electronic apparatus 1 in advance, this cosmetic cabinet list records a plurality of cosmetic product data respectively corresponding the cosmetic products owned by the user. Each of the plurality of the cosmetic product data may comprise the description data (such as name, brand, date of purchase, remaining capacity, usage history, etc. of the cosmetic product) and/or the appearance feature(s) (such as shape feature, color feature, bar code feature and so forth of the appearance of the cosmetic product) of the cosmetic product.

Then, the processing module 10 may perform the steps S104 and S109 in order or simultaneously.

Step: S104: the processing module 10 starts to measure the single round usage time of this cosmetic product being recognized.

Step: S 105: the processing module 10 recognizes the image of the user in the newly captured detection image, and determines whether the user stops using the cosmetic product recognized in the step S102 continuously.

One of the exemplary embodiments, in the step S102, the processing module 10 starts to detect the position(s) of the designated portion(s) of the user and the position(s) of the cosmetic product(s) being used continuously, and determines whether the user stops using the cosmetic product(s) based on the positions of the designated portion(s) and the cosmetic product(s) being used. For example, the processing module 10 may determine that the user stops using the cosmetic product when the cosmetic product separates from (do not touch) the user's hands or the cosmetic product goes away from the user's face.

One of the exemplary embodiments, the processing module 10 determines that the user stops using the cosmetic product when recognizing that the designated portion of the user separate from the cosmetic product for a default usage time (such as 5 seconds) or touch with another cosmetic product.

If the processing module 10 determines that the user stops using the cosmetic product being recognized, the step S106 will be performed. Otherwise, the processing module 10 performs the step S105 again for continuous detection.

Step: S106: the processing module 10 stops measuring the single round usage time, and configures the measure result as the usage time of this round of the cosmetic product data.

Then, the processing module 10 may perform the steps S107 and S109 in order or simultaneously.

Step: S107: the processing module 10 records the usage of the cosmetic product being stopped using in this round. Such as recording the single round usage time, the quantity used in this round, the usage times, the usage order, usage timing and/or so on in the corresponding cosmetic product data. The quantity used in this round could be estimated based on the single round usage time or image recognition technology, such as recognizing the remaining capacity of the cosmetic product by computer vision for estimating the quantity used in this round. For example, the processing module 10 may record that in this round, the cosmetic product data corresponding to the cosmetic product being stopped using is used when going out, at home or before sleep, or record which cosmetic product is used before or after the cosmetic product data corresponding to the cosmetic product being stopped using.

One of the exemplary embodiments, the processing module 10 may further update the accumulated usage time and the accumulated usage count (such as increasing once) of the cosmetic product data of the cosmetic cabinet list based on the single round usage time measured in the step S106.

One of the exemplary embodiments, the processing module 10 may further increase the accumulated usage count more than once (such as twice) if the single round usage time is too long, such as determining that the usage quantity is greater when the single round usage time is longer than a default usage time. Thus, the updated accumulated usage count may be close to express the used actual total usage of the cosmetic product.

Step: S108: the processing module 10 determines whether the electronic apparatus 1 is controlled to leave from the record mode. More specifically, when the user would like to finish the cosmetology procedure, the user may operate the electronic apparatus 1 to leave (or the electronic apparatus 1 leaves automatically by detection) the record mode for stopping recording the cosmetology procedure.

One of the exemplary embodiments, the user may input a finish cosmetology operation (such as pressing the record button of the input module 13 again) or a finish cosmetology voice (such as the user speaking a voice "finish cosmetology"), or pose a finish cosmetology gesture (such as the "5" gesture) to make the image capture module 12 capture the finish cosmetology gesture. Then, the processing module 10 controls the electronic apparatus 1 to leave from the record mode when detecting above designated input.

If the processing module 10 determines that the electronic apparatus leaves the record mode, the processing module 10 finishes the automatic recording method. Otherwise, the processing module 10 perform the step S102 again for detecting and recording the usage of the next cosmetic product.

Thus, the present disclosed example can actively recognize and record the usage amount of each cosmetic product and further manage the cosmetic product, so as to make the user focus on the cosmetology.

Besides, under the record mode, the processing module 10 may further control the electronic apparatus 1 to implement a reminder function of the incorrect procedure (steps S109-S110).

More specifically, when the cosmetic product currently used is recognized in the step S103, or the single round usage time is retrieved in the step S106, the processing module 10 may perform the step 109: the processing module 10 determining whether the usage of the cosmetic product is incorrect based on the standard cosmetology procedure pre-established.

One of the exemplary embodiments, each user may operate the electronic apparatus 1 to create one or more personally exclusive standard cosmetology procedure(s) in advance. For example, the standard cosmetology procedure when going out, the standard cosmetology procedure at home, the standard cosmetology procedure before sleep and so forth. These standard cosmetology procedures may be stored in the storage module 15.

One of the exemplary embodiments, each standard cosmetology procedure may record the usage way of each of the cosmetic products of the cosmetic cabinet list, such as the usage order, the usage timing (for example, being when going out, at home or before sleep), the single round usage time, the single usage quantity and so forth. The above way to use each of the cosmetic products may be recorded in the corresponding cosmetic product data. In the step S109, the processing module 10 is configured to determine whether the usage way of the cosmetic product data corresponding to the cosmetic product currently used by the user is consistent with the designated standard cosmetology procedure, such as the usage order is correct or the single round usage time is correct and so forth. Take cosmetology before sleep for example, the processing module 10 compares the usage way with the standard cosmetology procedure before sleep.

If the processing module 10 determines that the usage of the cosmetic product currently used is incorrect, the step S110 is performed. Otherwise, the processing module 10 performs step S108.

Step: S110: the processing module 10 outputs a reminder notification of incorrect procedure by the display module 11. Above reminder notification of incorrect procedure may be text, pattern or a combination of both, but this specific example is not intended to limit the scope of the present disclosed example.

One of the exemplary embodiments, the processing module 10 records the usage order or the usage timing of each of the cosmetic products used by the used under the record mode, and output the reminder notification of incorrect procedure by the display module 11 when the usage of the cosmetic product doesn't follow the standard usage order or the standard usage timing of the designated standard cosmetology procedure. Above reminder notification of incorrect procedure may be text, pattern or a combination of both.

One of the exemplary embodiments, the processing module 10 may remind the user the correct usage way of the cosmetic product currently used by the reminder notification of incorrect procedure.

One of the exemplary embodiments, the present disclosed example further a function of modifying procedure for making the user have the ability to modify the standard procedure when there is system misjudgment or the determination do not meet the user's expectation. More specifically, after watching the reminder notification of incorrect procedure, the user may input by the input module 13 an operation of modifying procedure or a voice of modifying procedure, or input by the image capture module 12 a gesture of modifying procedure. For example, replacing the usage way of the standard cosmetology procedure or the usage way recorded in the cosmetic product data with the current usage timing of the cosmetic product. Then, the processing module 10 may modify the standard cosmetology procedure based on the input, such as modifying the standard usage order or the standard usage timing of the standard cosmetology procedure based on the current usage order or the current usage timing of the cosmetic product.

Thus, the present disclosed example can prevent the user from the incorrect usage of the cosmetic product via actively detecting whether the current usage way of the cosmetic product is incorrect and issuing the reminder notification when it is incorrect.

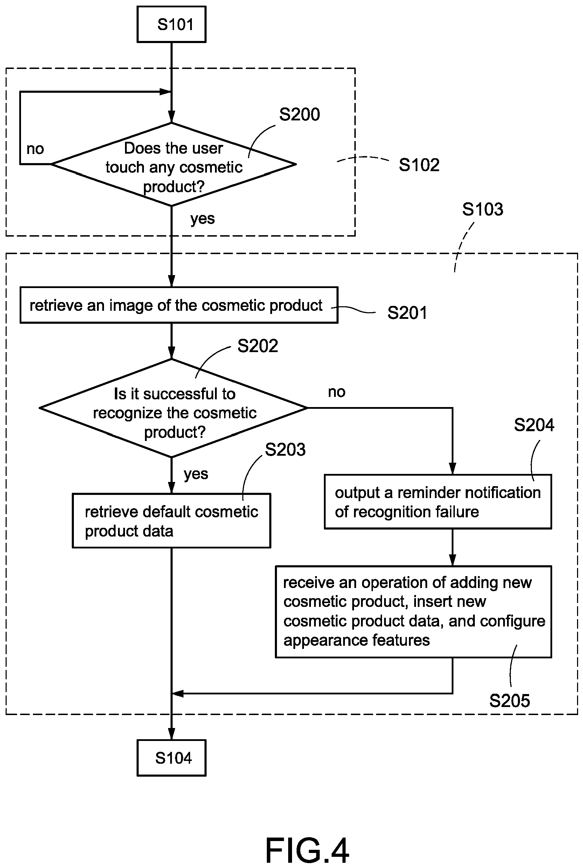

Please refer to FIG. 3 and FIG. 4 together. FIG. 4 is a partial flowchart of a method of automatically recording cosmetology procedure according to a second embodiment of the present disclosed example. Compare to the method of automatically recording cosmetology procedure shown in FIG. 3, the step S102 of the method of automatically recording cosmetology procedure of this embodiment may comprises step S200.

Step: S200: the processing module 10 determines that the user starts to use the cosmetic product when recognizing that the user touches any of the cosmetic products based on the detection images. Otherwise, the processing module 10 performs the step S200 again.

Moreover, in this embodiment, the step S103 may comprises steps S201-S205.

Step: S201: the processing module 10 crops the detection image for obtaining the appearance image of the cosmetic product being currently touched by the user from the detection image(s).

Step: S202: the processing module 10 compares the appearance image of the cosmetic product with each of the appearance feature(s) of each cosmetic product data to determine whether the cosmetic product being currently touched by the user is the cosmetic products corresponding to one the plurality of cosmetic product data recorded in the cosmetic cabinet list.

More specifically, each cosmetic product data of the cosmetic cabinet list may comprise the appearance feature(s) of the corresponding cosmetic product. Above appearance feature(s) may be the rules or values for recognition and obtained by analysis and process, so as to be used to express the appearance characteristics of the cosmetic product.

For example, above appearance features may be used to describe the shape, color(s) or a combination of both of the appearance of the cosmetic product.

In another example, the user may paste the designated text-pattern tag on the outside surface of each cosmetic product. Above text-pattern tag Thus, if there are cosmetic products with the appearances being similar as each other, via sticking the pattern-text tags which their appearances are significantly different from each other on the cosmetic products additionally, the present disclosed example can still effectively improve the success rate and correctness of recognition, and have the ability to determining the correct cosmetic product data.

In another example, each of above-mentioned appearance features may be used to describe the content of barcode of each cosmetic product, such as the sequence number obtained by decoding the barcode image.

If the processing module 10 determines that the cosmetic product currently used is consistent with the cosmetic product corresponding to any of the cosmetic product data of the cosmetic cabinet list, step S203 is performed.

Step: S203: the processing module 10 determines that the cosmetic product currently used is a known cosmetic product (such as one of the cosmetic products owned by the user and having been established), and loads the corresponding cosmetic product data (may comprise the description data and the appearance feature).

One of the exemplary embodiments, in the following steps, the processing module 10 may recognize and trace the appearance image of each cosmetic product in the screen based on the appearance feature of the loaded cosmetic product data, so as to improve the effectiveness and correctness of recognition.

One of the exemplary embodiments, the processing module 10 may output the description data (such as the product name, the recommended usage quantity, the accumulated usage time, the expiration date, etc.) of the loaded cosmetic product data on the display module 11 as the reference to the user.

step: S200: the processing module 10 determines that the cosmetic product currently used doesn't match with all of the cosmetic products respectively corresponding to all of the plurality of cosmetic product data, the processing module 10 performs step S204: the processing module 10 outputting a reminder notification of recognition failure on the display module 11 for reminding the user that the cosmetic product currently used is a new cosmetic product which its cosmetic product data has not been established.

Step: S205: the user may input by the input module 13 a receive operation of adding new cosmetic product after watching the reminder notification of recognition failure, in response, the processing module 10 inserts the cosmetic product data corresponding to the cosmetic product being unrecognizable in the cosmetic cabinet list. the above operation of adding new cosmetic product may comprise inputting the description data of the cosmetic product being unrecognizable.

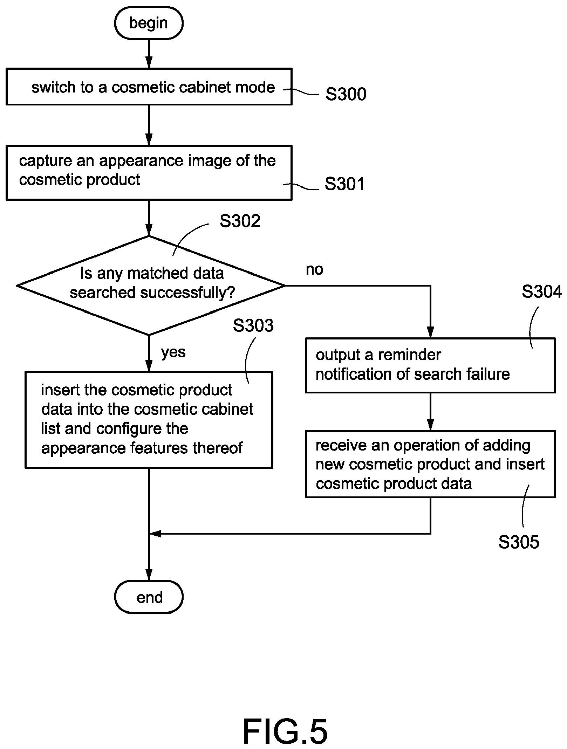

Then, the processing module 10 may configure the new cosmetic product data (may comprise the description data and the appearance feature) in the cosmetic cabinet list based on the operation of adding new cosmetic product (such as configuring according to the description data of the cosmetic product being unrecognizable). The detail of inserting the cosmetic product data is described in the following embodiment of FIG. 5.

Moreover, after the new cosmetic product data is established, the processing module 10 determines that the cosmetic product currently used (namely, the cosmetic product being unrecognizable previously) is the cosmetic product corresponding to the newly inserted cosmetic product data, and then the step S104 is performed.

Thus, the present disclosed example can effectively recognize the cosmetic product currently used even its a new cosmetic product.

Please refer to FIG. 5, which is a flowchart of adding cosmetic product data of a third embodiment of the present disclosed example. Compare to the automatic recording method shown in FIG. 3, the automatic recording method of this embodiment is provided to implement a cosmetic cabinet function for making the user convenient to view all of the cosmetic product owned by the user, and a function of adding new cosmetic product for assist the user to add the new cosmetic product data.

More specifically, the automatic recording method of this embodiment comprises the following steps used to implement the function of managing cosmetic products.

Step: S300: when the user would like to manage the cosmetic product data, the user may operate the electronic apparatus 1 (or the electronic apparatus 1 automatically switches by detection) to switch to a cosmetic cabinet mode.

One of the exemplary embodiments, the user may input by the input module 13 in operation of entering cosmetic cabinet (such as pressing a button of entering cosmetic cabinet mode cabinet) or a voice of entering cosmetic cabinet (such as the user speaking a voice "enter cosmetic cabinet" if the input module 13 is a microphone), or pose a gesture of entering cosmetic cabinet (such as the "O" hand gesture) to make the image capture module 12 capture the gesture. Then, the processing module 10 controls the electronic apparatus 1 to switch to the cosmetic cabinet mode when detecting the above-designated input.

Moreover, under the cosmetic cabinet mode, the processing module 10 may control the input module 13 and the display module 11 to interact with the user for providing all the cosmetic product data to the user for browsing.

Then, when the user would like to add any cosmetic product data to the cosmetic cabinet list, the user may input an operation of adding new cosmetic product, a voice of adding new cosmetic product or a gesture of adding new cosmetic product for making the processing module 10 to perform steps S301-S305 to adding the new cosmetic product data to the cosmetic cabinet list of the user.

Step: S301: the processing module 10 captures by the image capture module 12 an appearance image of the cosmetic product which the user would like to establish its profile.

For example, the image capture module 12 may be controlled to capture the barcode, pattern-text tag or the appearance of the cosmetic product. The appearance captured could be part (such as the product name or the appearance image(s) of part of angles of views) or whole (such as the appearance images of all of angles of views) of the cosmetic product.

Step: S302: the processing module 10 searches the sample data matched with the appearance image in the cosmetic product database based on the appearance image being captured.

One of the exemplary embodiments, above-mentioned cosmetic product database may be stored in the storage module 15 or the network server 3.

One of the exemplary embodiments, above-mentioned cosmetic product database stores a plurality of sample data (may comprise sample description data, such as product name, usage way, single round usage quantity and so forth) respectively corresponding to a plurality of different types of cosmetic products (namely, the sample cosmetic products) being common in the market.

One of the exemplary embodiments, above-mentioned cosmetic product database may further store the sample feature(s) of each sample cosmetic product corresponding to each sample data.

If the processing module 10 determines that the appearance image matches with the sample feature of each sample data, the processing module 10 performs step: S303: the processing module 10 inserts one cosmetic product data in the cosmetic cabinet list based on the sample description data of the matched sample data, such as configuring part or all of the sample description data as the description data of the cosmetic product data.

Moreover, the processing module 10 may configure the appearance feature of the inserted cosmetic product data further based on the appearance feature captured in the step S301 or the sample features of the sample data downloaded from the cosmetic product database.

Thus, via searching in the cosmetic product database, the present disclosed example can automatically finish the insertion of cosmetic product data, and saving the user operations.

If the processing module 10 determines that the appearance image doesn't match with all of the sample features of all of the plurality of sample data, the processing module 10 performs step: S304: the processing module 10 outputting by display module 11 a reminder notification of search failure for reminding the user the failure of automatically adding cosmetic product.

One of the exemplary embodiments, the processing module 10 may further interact with the user by the input module 13 and the display module 11 to guide the user to adding the new cosmetic product data manually.

More specifically, the user may input by the input module 13 an operation of adding new cosmetic product for inputting the description data of the cosmetic product which the user would like to add. Then, the processing module 10 executes step S305.

step: S305: the processing module 10 inserts the cosmetic product data to the cosmetic cabinet list based on the description data inputted by the operation of adding new cosmetic product.

One of the exemplary embodiments, the processing module 10 may further capture by the image capture module 12 one or more appearance images of the appearance of the cosmetic product which the user would like to add, and configure the appearance feature of the cosmetic product data based on the captured appearance images.

Thus, via providing the function of adding the new cosmetic product manually, the present disclosed example can obtain any cosmetic product data corresponding to any type of cosmetic products, recognize any type of cosmetic products, and implement the automatic record function.

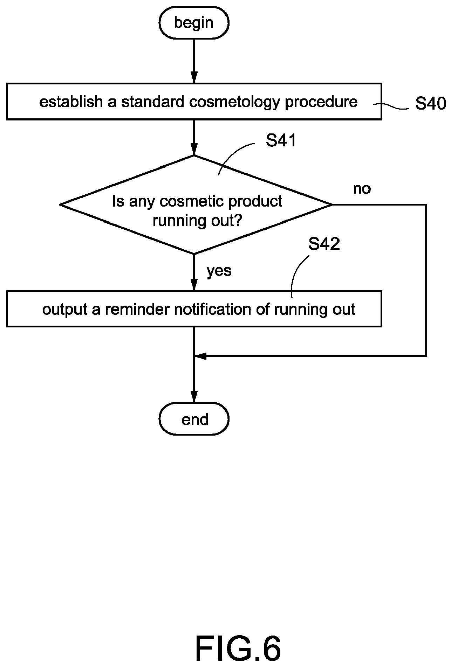

Please refer to FIG. 6, which is a flowchart of building standard cosmetology procedure and running out reminding of a fourth embodiment of the present disclosed example. The automatic recording method of this embodiment provides a function of establishing standard cosmetology procedure having the ability to generate the standard cosmetology procedure being suitable for the user by the user configuring manually or the automatic recording system configuring automatically. More specifically, the automatic recording method of this embodiment comprises following step S40 for implementing the function of establishing standard cosmetology procedure.

Step S40: the processing module 10 builds a standard cosmetology procedure.

One of the exemplary embodiments, the processing module 10 may receive an operation of building procedure of the user by the input module 13, and building the new standard cosmetology procedure based on the operation of building procedure, and configure the details of the standard cosmetology procedure, such as the cosmetic products being used, the usage order of the cosmetic products, the single round usage time, the single round usage quantity, the usage timing or the usage order of each cosmetic product or the execution timing of the standard cosmetology procedure.

One of the exemplary embodiments, the processing module 10 may automatically build a standard cosmetology procedure for the user exclusive use based on the records of the way in which the user uses each cosmetic product, the records are recorded many times when each time switching to the recording mode. Moreover, the processing module 10 may further configure the details of the standard cosmetology procedure based on the past record (or information in the network).

For example, if the user has the daily habit of using a first cosmetic product, a second cosmetic product and a third cosmetic product before sleep, the processing module 10 may configure above usage way as a standard cosmetology procedure before sleep for the user exclusive use, and above usage order may be recorded in this standard cosmetology procedure.

Besides, the automatic recording method of this embodiment further provides a function of reminding that cosmetic product is running out. The function of reminding that cosmetic product is running out has the ability to detect whether any cosmetic product is upcoming running out, and issues a reminder notification when determining that any cosmetic product is running out.

More specifically, the automatic recording method of this embodiment comprises following steps S41-S42 for implementing the function of managing cosmetic products.

Step: S41: the processing module 10 determines whether any of the cosmetic products is running out.

One of the exemplary embodiments, the processing module 10 may measure the accumulated usage time or accumulated usage count of each cosmetic product based on the usage record of the cosmetic product data corresponding to the cosmetic product, and determine that the corresponding cosmetic product is running out when the accumulated usage count is not less than an expected available count or the accumulated usage time is not short than an expected available time.

One of the exemplary embodiments, if the user had replaced the cosmetic product with the same type (or effect) of new one in the past when last time the cosmetic product is running out, above expected available time and expected available count may be obtained by measuring based on the accumulated usage time or the accumulated usage time of the above same type of new one replaced last time. For example, the processing module 10 may configure above accumulated usage time as the expected available time and/or above accumulated usage count as the expected available count.

Step: S42: the processing module 10 outputs a reminder notification of running out for reminding the user that this cosmetic product is running out.

Thus, the user can prepare a spare of the cosmetic product before it is running out, so as to prevent the cosmetology from failure caused by no remaining of the cosmetic product during cosmetology procedure.

Please be noted that although in above-mentioned embodiments, the automatic recording method is executed at the local side, but this specific example is not intended to limit the scope of the present disclosed example.

One of the exemplary embodiments, the present disclosed example executes the automatic recording method in combination with cloud technology. More specifically, the electronic apparatus 1 is only used to capture the images, receive operation and display information (such as the steps S100, S101, S108 and S110 shown in FIG. 3, the steps S200, S201, S204 and S205 shown in FIG. 4, the steps S300, S301, S304 and S305 shown in FIG. 5, and/or the steps S42 shown in FIG. 6), part or all of the others processing steps are performed by the processing module 30 and the storage module 35 of the cloud server 3.

Take the automatic recording method shown in FIG. 3 for example, after the electronic apparatus 1 performs the step S100 and S101, the electronic apparatus 1 may upload the captured detection images to the cloud server 3 continuously, then the processing module 30 of the cloud server 3 performs the steps S102-S107 and S109. If the cloud server 3 determines that the usage of the cosmetic product currently used is incorrect, the cloud server 3 may transfers the corresponding command (such as the reminder command of incorrect procedure) to the electronic apparatus 1 by network, so as to make the electronic apparatus 1 outputs the corresponding reminder notification (such as the reminder notification of incorrect procedure) by the display module 11.

## Claims

1. A method of automatically recording cosmetology procedure, the method being applied to a system of automatically recording cosmetology procedure, the system of automatically recording cosmetology procedure comprising an image capture module (12), a display module (11) and a processing module (10, 30), the method of automatically recording cosmetology procedure comprising following steps:
a) capturing detection images continuously by the image capture module (12) under a record mode;
b) recognizing a cosmetic product, retrieving cosmetic product data corresponding to the cosmetic product being recognized and starting to measure a single round usage time when determining that a user starts to use the cosmetic product based on the detection images at the processing module (10, 30);
c) stopping measuring the single round usage time and recording usage of the cosmetic product in this round when determining that the user stops using the cosmetic product based on the detection images;
d) outputting a reminder notification of incorrect procedure by the display module (11) when determining that there is incorrect usage of the cosmetic product based on a standard cosmetology procedure; and
e) performing the step b) to the step d) repeatedly until leaving from the record mode.

2. The method of automatically recording cosmetology procedure according to claim 1, wherein the system of automatically recording cosmetology procedure further comprises an input module (13); the method of automatically recording cosmetology procedure comprises a step f) performed before the step a): switching to the record mode when a start cosmetology operation or a start cosmetology voice is received by the input module (13) or a start cosmetology gesture is captured by the image capture module (12); the step e) is performed to leave from the record mode when a finish cosmetology operation or a finish cosmetology voice is received by the input module (13) or a finish cosmetology gesture is captured by the image capture module (12).

3. The method of automatically recording cosmetology procedure according to claim 1, wherein the system of automatically recording cosmetology procedure further comprises a storage module (15, 35), the storage module (15, 35) stores the plurality of cosmetic product data respectively corresponding to the cosmetic products, each of the plurality of cosmetic product data comprises at least one appearance feature of the corresponding cosmetic product; the step b) comprises following steps:
b1) determining that the user starts to use the cosmetic product when determining that the user touches the cosmetic product based on the detection images;
b2) cropping the detection image for obtaining an appearance image of the cosmetic product touched by the user;
b3) comparing the appearance image of the cosmetic product with the appearance feature of each of the plurality of cosmetic product data to determine the cosmetic product data corresponding to the cosmetic product currently used; and
b4) starting to measure the single round usage time.

4. The method of automatically recording cosmetology procedure according to claim 3, wherein the system of automatically recording cosmetology procedure further comprises an input module (13), the step b) further comprises following steps:
b5) outputting a reminder notification of recognition failure by the display module (11) when the appearance image of the cosmetic product is not consistent with the appearance features of all of the plurality of cosmetic product data;
b6) receiving an operation of adding new cosmetic product by the input module (13), wherein the operation of adding new cosmetic product is to input description data of the cosmetic product failing to recognize; and
b7) configuring the cosmetic product data corresponding to cosmetic product failing to recognize based on the description data.

5. The method of automatically recording cosmetology procedure according to claim 3, wherein the step c) is performed to determine that the user stops using the cosmetic product when determining that the user stops touching the cosmetic product for a default usage time or touches another cosmetic product based on the detection images.

6. The method of automatically recording cosmetology procedure according to claim 3, wherein the step c) is performed to update an accumulated usage time or an accumulated usage count based on the single round usage time.

7. The method of automatically recording cosmetology procedure according to claim 3, wherein the storage module (15, 35) further stores a cosmetic cabinet list, the cosmetic cabinet list is used to record the plurality of cosmetic product data corresponding to the cosmetic products owned by the user, the method of automatically recording cosmetology procedure further comprises following steps:
g1) under a cosmetic cabinet mode, capturing an appearance image of the cosmetic product by the image capture module (12);
g2) comparing the appearance image with data in a cosmetic product database for searching sample data corresponding to a sample cosmetic product matched which its appearance matches with the appearance image, wherein the sample data comprises sample description data of sample the cosmetic product;
g3) inserting the cosmetic product data into the cosmetic cabinet list based on the sample description data of the sample cosmetic product; and
g4) configuring the appearance feature of the cosmetic product data based on the appearance feature or a sample feature of the sample data.

8. The method of automatically recording cosmetology procedure according to claim 7, further comprising following steps after the step g1):
g5) outputting a reminder notification of search failure of sample by the display module (11) when there is not any of the matched sample data being searched in the cosmetic product database;
g6) receiving an operation of adding new cosmetic product by the input module (13), wherein the operation of adding new cosmetic product is to input description data of the cosmetic product failing to add automatically;
g7) inserting the cosmetic product data corresponding into the cosmetic product to the cosmetic cabinet list based on the description data; and
g8) capturing a plurality of appearance images of the cosmetic product by the image capture module (12), and configuring the appearance feature of the cosmetic product being added based on the appearance images.

9. The method of automatically recording cosmetology procedure according to claim 1, wherein the standard cosmetology procedure comprises a standard usage order or a standard usage timing of the cosmetic products, the step d) is performed to record a usage order or a usage timing of each of the cosmetic products under the record mode, and output the reminder notification of incorrect procedure when the usage of the cosmetic product doesn't follow the standard usage order or the standard usage timing.

10. The method of automatically recording cosmetology procedure according to claim 9, wherein the system of automatically recording cosmetology procedure further comprises an input module (13), the method of automatically recording cosmetology procedure further comprises a step h) performed after the step d): modifying the standard usage order or the standard usage timing of the cosmetic product in the standard cosmetology procedure based on the current usage of the cosmetic product when receiving an operation of modifying procedure or a voice of modifying procedure by the input module (13), or capturing a gesture of modifying procedure by the image capture module (12).

11. The method of automatically recording cosmetology procedure according to claim 1, further comprising following steps:
i1) building the standard cosmetology procedure based on a usage record in the multiple rounds of each of the cosmetic products; or
i2) receiving an operation of building procedure by the input module (13) of the system of automatically recording cosmetology procedure, and building the standard cosmetology procedure based on the operation of building procedure, wherein the operation of building procedure is to input a usage order or a usage timing of the cosmetic products.

12. The method of automatically recording cosmetology procedure according to claim 1, wherein the system of automatically recording cosmetology procedure further comprises a storage module (15, 35), the storage module (15, 35) stores a cosmetic cabinet list, the cosmetic cabinet list is used to record the plurality of cosmetic product data corresponding to the cosmetic products owned by the user, the method of automatically recording cosmetology procedure further comprises a step j) outputting a reminder notification of running out by the display module (11) when determining that an accumulated usage count of any cosmetic product data is not less than an expected available count of the cosmetic product data or an accumulated usage time of any cosmetic product data is not shorter than an expected available time of the cosmetic product.

13. The method of automatically recording cosmetology procedure according to claim 1, wherein the system of automatically recording cosmetology procedure further comprises an electronic apparatus (1), the image capture module (12), the display module (11) and the processing module (10, 30) are arranged in the electronic apparatus (1);
the step a) is to capture the detection images continuously by the image capture module (12) under the record mode, execute a mirroring flip process on the detection images and display the detection images being flipped by the display module (11) instantly for showing an electronic mirror image.

14. The method of automatically recording cosmetology procedure according to claim 1, wherein the system of automatically recording cosmetology procedure further comprises an electronic apparatus (1), electronic apparatus (1) comprises a mirror (16) and a case, the mirror (16) is used to show an optic mirror image, the image capture module (12), the display module (11) and the processing module (19, 30) are arranged in the electronic apparatus (1), the display module (11) is arranged in the case and rear of the mirror (16), a display surface of the display module (11) towards front of the mirror (16), the image capture module (12) shoots towards front of the mirror (16);
the step d) is performed to show the reminder notification of incorrect procedure on the mirror (16) and display the optical mirror image simultaneously on the mirror (16) by transmission.

15. The method of automatically recording cosmetology procedure according to claim 1, wherein the system of automatically recording cosmetology procedure further comprises an electronic apparatus (1) and a cloud server (3), the electronic apparatus (1) comprises the image capture module (12), the display module (11) and a transmission module (14), the electronic apparatus (1) is connected to the cloud server (3) by connecting to a network (2) by the transmission module (14), the processing module (10, 30) is arranged in the cloud server (3);
The step a) is performed to capture the detection images continuously at the electronic apparatus, and upload the detection images to the cloud server (3);
the step b) and the step c) are performed by the cloud server (3);
the step d) is performed to transfer a reminder notification of incorrect procedure to the electronic apparatus at the cloud server (3) for making the electronic apparatus (1) to output the reminder notification of incorrect procedure when receiving a reminder comment of incorrect procedure.
